# EUROPÄISCHE PATENTSCHRIFT

(11) **EP 0 406 375 B1**
(45) Veröffentlichungstag und Bekanntmachung des Hinweises auf die Patenterteilung: **01.03.1995**
(21) Anmeldenummer: 90900864.1
(22) Anmeldetag: 08.12.1989
(51) Int. Cl.: A61F 2/02

(54) **ALLOPLASTISCHES IMPLANTAT**
ALLOPLASTIC IMPLANT
IMPLANT ALLOPLASTIQUE

(30) Priorität: 08.12.1988 DE 3841401
(43) Veröffentlichungstag der Anmeldung: 09.01.1991
(73) Patentinhaber: LEMPERLE, Martin, D-60314 Frankfurt (DE)
(72) Erfinder: LEMPERLE, Martin, D-60314 Frankfurt (DE)
(74) Vertreter: Jaeger, Klaus, Dr.
(86) Internationale Anmeldenummer: EP8901508
(87) Internationale Veröffentlichungsnummer: WO9006093

(56) Entgegenhaltungen:
- WO-A-87/07495
- WO-A-89/06944
- DE-A- 2 812 696
- FR-A- 2 287 210
- US-A- 4 197 846
- US-A- 4 380 569
- US-A- 4 500 658
- US-A- 4 657 548

## Beschreibung

Die Erfindung betrifft ein alloplastisches Implantat auf Basis eines gewebeverträglichen Feststoffes. Dieses Implantat dient insbesondere zum Ausgleich von Hautunebenheiten, kann jedoch auch für andere Zwecke in der plastischen Chirurgie eingesetzt werden.

Es wird seit langer Zeit und auf vielfache Weise versucht, pathologisch veränderte oder durch Unfall zerstörte Elemente des menschlichen Körpers mit Hilfe chirurgischer Maßnahmen "wiederherzustellen" und erforderlichenfalls durch Fremdkörper zu ersetzen. Dabei finden insbesondere auf dem Gebiete der plastischen Chirurgie und der Schönheitschirurgie Implantate immer mehr Anwendung.

Häufig besteht der Wunsch danach, Hautunebenheiten jeglicher Genese dauerhaft und ohne Nebenwirkungen auszugleichen. Für diesen Zweck und natürlich auch für andere Zwecke der plastischen Chirurgie hat man bereits verschiedene Stoffe eingesetzt.

So hat man beispielsweise sehr häufig Kollagen zur Anwendung gebracht. Kollagen ist aber ein xenogenes Eiweißprodukt, das im Körper abgebaut wird. Hat man beispielsweise eine Hautunebenheit mit Kollagen ausgeglichen, beispielsweise durch eine Injektion unter die Haut (intra- oder subkutan), dann sind in der Regel Nachinjektionen erforderlich, um das ursprünglich erzielte Ergebnis der ersten Injektion wiederherzustellen.

Kollagen wirkt außerdem allergisierend, so daß es in mindestens 3 % der Fälle zu allergischen Erscheinungen kommt. Dieses Phänomen ist insbesondere dann ausgeprägt, wenn mehrere Injektionen für die Erlangung des erwünschten Ergebnisses erforderlich sind. Letzteres ist in der Regel der Fall.

Es ist auch bekannt, für den oben genannten Zweck Gelatine zum Einsatz zu bringen. Gelatine besitzt jedoch ähnliche Nachteile wie Kollagen und ist darüber hinaus wesentlich schlechter injizierbar.

Auch Silicon bzw. Siliconöle haben bereits Anwendung gefunden. Siliconöle bewirken jedoch eine ausgeprägte Gewebsreaktion am Ort der Injektion (Siliconom). Die Siliconöle werden zudem am Injektionsort nur schlecht vom Gewebe fixiert, so daß es in der Folge zu einer Verschleppung des injizierten Siliconöls in nahe oder entferntere Körperteile kommt. Dazu zählen Lymphknoten und Leber.

Ein Implantat, insbesondere zur Brustvergrößerung, ist aus der FR-A-2 287 210 bekannt. Dieses Implantat besteht aus einem weichen Säckchen aus einem gewebeverträglichen Material, insbesondere festem Siklicon. Die Abmessungen dieses Implantats müssen je nach Erfordernis gewählt werden.

Auch ist es bekannt, für die hier in Rede stehenden Zwecke eine Fettunterspritzung mit körpereigenem Fett vorzunehmen. Es hat sich jedoch herausgestellt, daß eine derartige Maßnahme häufig zu Infektionen führt. Zudem wird das körpereigene Fett schnell und stark vom Körper resorbiert, so daß in der Regel nur 10 % des eingespritzten Fettes ortsfest an der gewünschten Stelle verbleiben.

Aufgabe der vorliegenden Erfindung ist es, ein alloplastisches Implantat zur Verfügung zu stellen, das einfach injizierbar ist, an dem eingespritzten Ort ortsfest verbleibt und gewebeverträglich ist, so daß keine Nebenwirkungen hervorgerufen werden.

Gelöst wird diese Aufgabe durch die Lehre des Anspruchs 1.

Erfindungsgemäß wird somit ein gewebeverträglicher und als Pulver vorliegender Feststoff als Implantat eingesetzt. Die diesen Feststoff bildenden Feststoffpartikel werden beispielsweise mit Hilfe einer Injektionsspritze und gegebenenfalls in ein Schwebemittel inkorporiert an die gewünschte Stelle injiziert bzw. gespritzt.

Die erfindungsgemäß zum Einsatz gebrachten Feststoffpartikel besitzen eine glatte Oberfläche und sind frei von Ecken, Kanten etc. Mit anderen Worten, die Feststoffpartikel dürfen an ihrer Oberfläche keinen scharfen Übergänge aufweisen, wie dies beispielsweise bei Kanten und Ecken der Fall ist. Natürlich dürfen auch keine irgendwie gearteten Spitzen bzw. spitzartigen Vorsprünge vorhanden sein. Auch sollte die Oberfläche möglichst porenfrei sein.

Bei den erfindungsgemäß eingesetzten Feststoffpartikeln muß der Übergang von einer Außenfläche zur anderen somit kontinuierlich erfolgen. Gegebenenfalls vorhandene Übergänge, wie beispielsweise an den Kanten eines Würfels, müssen abgerundet sein.

Erfindungsgemäß können somit keine Feststoffpartikel eingesetzt werden, bei denen es sich um Kristallite (zum Beispiel nadelförmige) oder um durch mechanische Zerkleinerung (z. B. Mahlen) von größeren Einheiten erhaltene Partikel handelt, denn derartige Partikel besitzen eben die oben erwähnten, scharfen Ecken und Kanten.

Durch diese glatte und abgerundete Oberflächenstruktur werden keine Zellen oder andere Gewebsstrukturen verletzt. Zudem wird die Gefahr von Gewebsreaktionen mit nachfolgender Entzündung minimiert.

Vorzugsweise setzt man rotationssymmetrische und insbesondere elipsoide oder kugelförmige Feststoffpartikel ein. Auch ist es möglich, Feststoffpartikel unterschiedlicher geometrischer Form zur Anwendung zu bringen, sofern alle diese Partikel eine glatte und abgerundete Oberfläche besitzen.

Die Feststoffpartikel, die ja als Pulver bzw. Staub vorliegen, besitzen einen durchschnittlichen Durchmesser von 10 »m bis 200 »m. Derartige Feststoffpartikel sind zu groß, um von Monozyten "gefressen" zu werden.

Die Feststoffpartikel haben zweckmäßigerweise einen mittleren Durchmesser von ungefähr 15 bis ungefähr 200 »m und insbesondere bevorzugt von ungefähr 15 bis ungefähr 60 »m.

In diesem Fall sind die Feststoffpartikel auch klein genug, um durch die Kanüle einer Injektionsspritze an den gewünschten Ort gebracht werden zu können.

Partikel mit den genannten Durchmessern können auch nicht als einzelne Fremdkörper in bzw. unter der Haut ertastet werden.

Die Feststoffpartikel besitzen vorzugsweise einen solchen Durchmesser, daß sie am Einsatzort nicht durch Lymph- oder sonstige Gewebsbahnen abgeschwemmt werden.

Bei Feststoffpartikeln, die keine Kugeln darstellen, wird im übrigen im Rahmen der hier vorliegenden Unterlagen unter Durchmessser der größte Durchmesser der kleinsten Querschnittsfläche verstanden.

Die eingesetzten Partikel werden aufgrund ihrer Form, Oberfläche und Größe von den körpereigenen Freßzellen (Makrophagen) nicht als Fremdkörper erkannt, so daß keine Abwehrreaktionen auftreten.

Ein Vorteil des Einsatzes von Partikeln in Kugelform oder kugelähnlicher Form besteht darin, daß sie am Einsatzort eine dicht gepackte Anordnung bilden können.

Die erfindungsgemäß eingesetzten Feststoffpartikel bestehen aus einem inerten, gewebeverträglichen Material. Bei diesem Material kann es sich beispielsweise um Glas handeln, welches in Form von Glaskügelchen mit glatter und abgerundeter Oberfläche vorliegt.

Vorzugsweise bestehen die erfindungsgemäß eingesetzten Feststoffpartikel aus einem Kunststoff und insbesondere aus einem völlig ausgehärteten bzw. auspolymerisierten Kunststoff, so daß keine potentiell toxischen und kanzerogenen Restmonomere in den Körper der behandelten Person gelangen können.

Im Prinzip kann jeder inerte gewebeverträgliche Kunststoff für die Herstellung der erfindungsgemäß zum Einsatz gebrachten Kunststoffpartikel verwenden werden. Vorzugsweise setzt man jedoch als Kunststoff Polymethacrylate und insbesondere Polymethylmethacrylat (PMMA) ein.

Auspolymerisiertes PMMA ist körperverträglich und kann unbedenklich in den menschlichen Körper eingesetzt werden, so daß es als chemisch und physikalisch inert bezeichnet werden kann. Aus diesem Grunde wurde dieser Kunststoff auch bereits vielfach zur Herstellung von Implantaten verwendet, beispielsweise zur plastischen Deckung von Knochendefekten in Gesicht und Schädel oder als Gelenkplastik. Dieser Kunststoff wird zudem auch zur Herstellung künstlicher Zähne, als Nahtmaterial, für die Herstellung von Intraokularlinsen und von Dialysemembranen eingesetzt.

Um die erfindungsgemäß zum Einsatz gebrachten Feststoffpartikel bzw. Kunststoffpartikel als Implantat in und unter die Haut spritzen zu können, schlämmt man diese Partikel vorzugsweise in einer Art Schwebemittel auf. Als Schwebemittel kann man beispielsweise Wasser, Alkohole, insbesondere Ethylalkohol sowie Mischungen daraus verwenden.

Die erfindungsgemäß eingesetzten Schwebemittel enthalten zweckmäßigerweise auch ein Tensid, beispielsweise Tween 80, da ein derartiges Tensid die Oberflächenspannung des Wassers verändert, so daß die Feststoffpartikel und insbesondere die Kunststoffpartikel besser schweben.

Das Mischungsverhältnis der Komponenten des Schwebemittels kann man nach den Bedürfnissen und insbesondere nach der Größe der für die Injektion eingesetzten Spritze wählen. Als zweckmäßig hat sich eine Mischung aus 0,5 ml Ethylalkohol, 0,5 ml Tween 80 und 9 ml Wasser herausgestellt. Die Bezeichnung "Tween" ist im übrigen ein Warenzeichen der ICI Americas Inc. Unter dieser Bezeichnung werden Polyoxyethylenderivate der Sorbitanester vertrieben. Beim Tween 80 handelt es sich um Polyethoxysorbitanoleat. Nicht nur die erwähnte Tween-Type (Tween 80), sondern auch andere Tween-Typen sind für die erfindungsgemäßen Zwecke einsetzbar.

Zur Applikation bzw. Injektion der erfindungsgemäß eingesetzten Feststoffpartikel schlämmt man somit diese in einem flüssigen inerten Medium auf. Als vorteilhaft hat sich ein Verhältnis von zwei Volumenteilen Schwebemittel und ein Volumenteil Feststoffpartikel bzw. Kunststoffkügelchen herausgestellt.

Vorzugsweise setzt man ein an sich bekanntes, im Körper abbaubares Gel, beispielsweise auf Basis von Gelatine, als Schwebemittel ein.

Durch den Einsatz eines Schwebemittels ist es leichter, die erfindungsgemäß zum Einsatz gebrachten Feststoffpartikel mit Hilfe einer Injektionsspritze z.B. intracutan zu injizieren. Für eine derartige Injektion kann man beispielsweise 20 G - 27 G Kanülen einsetzen.

Um zu untersuchen, welche Gewebsreaktionen nach Einbringen des erfindungsgemäß eingesetzten körperfremden Materials auftreten, wurden Tierversuche durchgeführt.

Für die Untersuchungen wurden PMMA-Kügelchen mit glatter, abgerundeter Oberfläche und mit verschiedenen Durchmessern, beispielsweise von 40 bis 80 »m sowie von 15 bis 60 »m, eingesetzt. Als Schwebemittel wurde eine Mischung aus 0,5 ml Ethylalkohol, 0,5 ml Tween und 9 ml Wasser eingesetzt. Derartige PMMA-Kügelchen sind an sich bekannt und im Handel erhältlich.

Vor der Injektion wurde dieses Schwebemittel, welches dafür sorgt, daß die kleinen Kügelchen in der Injektionsspritze bedingt durch die Gravitation nicht absinken und damit eine Injektion unmöglich machen, mit den PMMA-Kügelchen vermengt, und zwar in einem Verhältnis von etwa 1 Vol.-Teil Kügelchen und 2 Vol.-Teilen Schwebemittel.

Dieses erfindungsgemäße alloplastische Implantat wurde männlichen Wistar-Ratten mit einem Gewicht von 200 bis 250 g in Vollnarkose mit Nembutal an vier Stellen in einer Menge von 0,5 ml mit Hilfe einer 20 G Kanüle intracutan in die Bauchhaut injiziert. Es wurde bewußt die Bauchseite der Tiere genommen, da diese im Verhältnis zur Rückenseite wesentliche zarter und flexibler ist. Nach der Injektion bildeten sich durch das injizierte Volumen kleine Quaddeln an der Injektionsstelle. Dies war jedoch auch der Fall, wenn das Schwebemittel allein injiziert wurde.

Insgesamt wurde 39 Tieren das erfindungsgemäße alloplastische Implantat injiziert. Nach der Injektion wurden im Abstand von 3,6,9,12,15,21 und 28 Tagen sowie 8,12,16,20,24 und 28 Wochen je drei der Versuchstiere getötet. Die Bauchhaut wurde dann mittels Enthaarungscreme rasiert. An den Injektionsstellen wurden die noch sichtbaren, allerdings nicht mehr erhabenen Kügelchen mit einem Teil der Bauchhaut exzidiert und untersucht.

Die Ergebnisse der durchgeführten mikroskopischen und makroskopischen Untersuchungen lassen sich wie folgt zusammenfassen.

Innerhalb der Versuchszeit von 28 Wochen starb keines der Versuchstiere. Nach der Injektion traten keine über das bei Verletzungen (Einstich der Kanüle) physiologische Maß hinausgehende Abwehrreaktionen bzw. Entzündungen auf. Auch in der darauf anschließenden Zeit konnte keine derartige Reaktion beobachtet werden. Es fanden sich keine Tumore, weder im Bereich der Injektion, noch im Gesamtorganismus. Die Bauchhaut zeigte äußerlich keine pathologischen Veränderungen. Innerhalb weniger Tage war bereits wieder ein Fellwachstum zu erkennen. Die angrenzenden Lymphgefäße und Lymphknoten waren ebenfalls ohne pathologischen Befund.

Nach der Injektion der eingesetzten PMMA-Kügelchen wandern während der ersten drei Tage Monozyten in den Injektionsbereich.

Anschließend findet eine Differenzierung der Monozyten statt. Die verschiedenen Differenzierungsformen sind ca. nach 6 d nach der Applikation der Fremdkörper erkennbar. Man kann Makrophagen, Fremdkörper-Riesenzellen und Fibrozyten erkennen. Dieser Differenzierungsprozeß erstreckt sich bis etwa zu 16. Woche nach dem Einbringen des erfindungsgemäßen Implantats. Der Differenzierungsprozeß beschränkt sich jedoch im wesentlichen auf die Fibrozyten, die durch eine weitere Umwandlung in Fibrinfasern die PMMA-Kügelchen gegen den tierischen Körper abschirmen und dem Kugelhaufen dadurch sowohl nach innen als auch nach außen eine gewisse physikalische Stabilität verleihen.

Eine Phagozytierung bzw. Lysierung der PMMA-Kügelchen kann aufgrund deren Größe und physikalischen Stabilität nicht stattfinden. Der Tierkörper kann daher nur die Fremdkörper fibrotisch abkapseln, um diese Fremdkörper zu "beseitigen". Ein derartiger Prozeß findet bei nahezu jedem Fremdkörper statt, den der tierische Körper ansonsten nicht zerstören kann.

Die fibrotische Bindegewebsvermehrung ist eine natürliche Reaktion auf die durch die Injektionskanüle hervorgerufene Verletzung des Gewebes. Diese fibrotische Reaktion kommt aufgrund der glatten Oberfläche und der chemischen Inertheit der PMMA-Kügelchen schon nach wenigen Wochen völlig zum Erliegen. Ab dann bleiben die Kügelchen reaktionslos im Gewebe liegen.

Zusammenfassend läßt sich feststellen, daß die intrakorporal eingebrachten PMMA-Kügelchen in eine zarte Bindegewebskapsel abgekapselt werden bzw. in Bindegewebsfasern eingebettet werden und im Gewebe ortsfest verbleiben. Die histologisch nachgewiesene Fremdkörperreaktion war minimal und auch durch die mechanische Verletzung des Gewebes bei der Injektion bedingt.

Im übrigen ist der Einsatz eines wie oben beschriebenen Schwebemittels nicht unbedingt erforderlich, denn die oben beschriebenen PMMA-Kügelchen können auch ohne Schwebemittel in den Körper eingebracht werden.

Um die Reaktion der Versuchstiere auf die Einbringung von PMMA-Kügelchen ohne Schwebemittel zu untersuchen, wurde die Bauchhaut der Versuchstiere mit Hilfe einer Stichinzision eröffnet. Anschließend wurde das PMMA-Pulver in die Öffnung eingestreut. Die Wunde wurde dann durch eine Einzelknopfnaht verschlossen.

Auch hier konnte der gleiche Reaktionsablauf wie bei den weiter oben näher beschriebenen Untersuchungen festgestellt werden. Es bildete sich eine zarte Bindegewebskapsel, welche die PMMA-Kügelchen einschloß.

Allerdings dürfte für eine intracutane oder subcutane Unterspritzung der Haut eines Menschen zum Ausgleich von Hautunebenheiten die Verwendung eines Schwebemittels angezeigt sein, da in diesem Fall das erfindungsgemäße Implantat mit Hilfe einer Spritze leichter injiziert werden kann.

Um dafür Sorge zu tragen, daß die eingesetzten Kügelchen besser im Schwebemittel schweben, kann man im übrigen auch das eingangs beschriebene Tensid dadurch ersetzen, daß man die Kügelchen gleichsinnig auflädt .Dadurch werden diese voneinander abgestoßen und schweben besser im Medium des Schwebemittels, das in diesem Fall ausschließlich aus Wasser, Alkohol oder aus einem Gemisch davon bestehen kann.

Bei PMMA-Kügelchen kann man diese gleichsinnige Aufladung der Kügelchen dadurch herbeiführen, daß man die bereits ausgehärteten Kügelchen nochmals "anschmilzt" und dann in einem elektrischen Feld auflädt.

## Patentansprüche

1. Alloplastisches Implantat auf Basis eines gewebeverträglichen Feststoffes,
dadurch **gekennzeichnet**,
daß der Feststoff pulverförmig ist und aus Feststoffpartikeln mit einem Durchmesser von durchschnittlich 10 »m bis 200 »m aufgebaut ist, die eine glatte sowie von Ecken und Kanten freie Oberfläche besitzen.

2. Implantat nach Anspruch 1,
dadurch **gekennzeichnet**,
daß die Feststoffpartikel rotationssymmetrisch und insbesondere elipsoid oder kugelförmig sind.

3. Implantat nach Anspruch 1 oder 2,
dadurch **gekennzeichnet**,
daß die Feststoffpartikel einen Durchmesser von 15 bis 60 »m aufweisen.

4. Implantat nach mindestens einem der Ansprüche 1 bis 3,
dadurch **gekennzeichnet**,
daß der Feststoff ein Kunststoff, insbesondere ein ausgehärteter Kunststoff ist.

5. Implantat nach Anspruch 4,
dadurch **gekennzeichnet**,
daß der Kunststoff ein Polymethacrylat, insbesondere Polymethylmethacrylat, ist.

6. Implantat nach mindestens einem der Ansprüche 1 bis 5,
dadurch **gekennzeichnet**,
daß die Feststoff- bzw. Kunststoffpartikel in einem physiologisch verträglichen Schwebemittel vorliegen.

7. Implantat nach Anspruch 6,
dadurch **gekennzeichnet**,
daß das Schwebemittel ein im Körper abbaubares Gel, insbesondere auf Basis von Gelatine, ist.

8. Implantat nach Anspruch 6
dadurch **gekennzeichnet**,
daß das Schwebemittel flüssig ist und insbesondere aus Wasser oder einem Alkohol, insbesondere Ethylalkohol, oder aus einer Mischung daraus besteht und ggf. mit einem Tensid versetzt ist.

9. Verwendung von pulverförmigen Feststoffpartikeln gemäß einem der Ansprüche 1 bis 8 als alloplastisches Implantat, insbesondere zum Ausgleich von Hautunebenheiten.

## Claims

1. Alloplastic implant based on a histocompatible solid, characterised in that the solid is in powder form and is composed of solid particles with a diameter of 10 to 200 »m on average and having a smooth surface free from corners and edges.

2. Implant according to claim 1, characterised in that the solid particles are rotationally symmetrical and have in particular an elliptical or spherical form.

3. Implant according to claim 1 or 2, characterised in that the solid particles have a diameter of 15 to 60 »m.

4. Implant according to at least one of claims 1 to 3, characterised in that the solid is a plastics, in particular a cured plastics.

5. Implant according to claim 4, characterised in that the plastics is a polymethacrylate, in particular polymethylmethacrylate.

6. Implant according to at least one of the claims 1 to 5, characterised in that the solid particles or plastics particles, respectively, are present in a physiologically acceptable suspending agent.

7. Implant according to claim 6, characterised in that the suspending agent is a gel, degradable in the body, in particular on the basis of gelatine.

8. Implant according to claim 6, characterised in that the suspending agent is liquid and consists in particular of water or an alcohol, in particular ethylalcohol, or of a mixture thereof and is optionally admixed with a tenside.

9. Use of the powderlike solid particles according to one of claims 1 to 8 as an alloplastic implant, more particularly to even out irregularities of the skin.

## Revendications

1. Implant alloplastique à base d'un solide histocompatible, caractérisé en ce que le solide est pulvérulent et est constitué de particules solides de diamètre compris en moyenne entre 10 »m et 200 »m, qui possèdent une surface lisse et exempte d'angles et d'arêtes.

2. Implant selon la revendication 1, caractérisé en ce que les particules solides sont à symétrie de révolution et notamment ellipsoïdales ou sphériques.

3. Implant selon la revendication 1 ou 2, caractérisé en ce que les particules solides présentent un diamètre compris entre 15 et 60 »m.

4. Implant selon au moins une des revendications 1 à 3, caractérisé en ce que le solide est une matière plastique, notamment une matière plastique totalement durcie.

5. Implant selon la revendication 4, caractérisé en ce que la matière plastique est un polyméthacrylate, notamment un poly(méthacrylate de méthyle).

6. Implant selon au moins une des revendications 1 à 5, caractérisé en ce que les particules solides ou de matière plastique sont présentes dans un agent de suspension physiologiquement toléré.

7. Implant selon la revendication 6, caractérisé en ce que l'agent de suspension est un gel biodégradable, notamment à base de gélatine.

8. Implant selon la revendication 6, caractérisé en ce que l'agent de suspension est liquide et se compose notamment d'eau ou d'un alcool, notamment l'alcool éthylique, ou d'un de leurs mélanges et qu'un agent tensioactif lui est ajouté le cas échéant.

9. Utilisation de particules solides pulvérulentes selon une des revendications 1 à 8 comme implant alloplastique, notamment pour compenser les inégalités cutanées.
